(19) Europäisches Patentamt

European Patent Office

Office européen des brevets

(11) EP 1 044 700 A2

(12) **EUROPEAN PATENT APPLICATION**

(43) Date of publication:
**18.10.2000   Bulletin 2000/42**

(51) Int Cl.⁷: **A61M 16/01**

(21) Application number: **00660068.8**

(22) Date of filing: **13.04.2000**

| | |
|---|---|
| (84) Designated Contracting States:<br>**AT BE CH CY DE DK ES FI FR GB GR IE IT LI LU MC NL PT SE**<br>Designated Extension States:<br>**AL LT LV MK RO SI** | (72) Inventor: **Järnefelt, Gustaf**<br>**02170 Espoo (FI)** |
| (30) Priority: **15.04.1999  FI  990843** | (74) Representative:<br>**Valkeiskangas, Tapio Lassi Paavali et al**<br>**Oy Kolster Ab,**<br>**Iso Roobertinkatu 23,**<br>**P.O. Box 148**<br>**00121 Helsinki (FI)** |
| (71) Applicant: **INSTRUMENTARIUM CORPORATION**<br>**00510 Helsinki (FI)** | |

(54) **Anesthesia machine**

(57)     A method and an arrangement in connection with anesthesia equipment comprising a gas mixer (1), a connecting stud (4) having a flow connection with the gas mixer (1), and a vaporizer (2) to be arranged in the connecting stud (4) for vaporizing an anesthetic, whereby the vaporizer (2) is equipped with a mechanical regulator (6) in order to regulate the set value of the vaporization. In order to achieve a flexible solution, a device (7) equipped with a flow measuring unit is arranged to measure the flow of the vaporizer (2). A reference scale (8) corresponding to the reference scale of the vaporizer is arranged in a moving regulating member of a mechanical regulator (6). The arrangement is equipped with a reader device (9) arranged to read the position of the reference scale (8), and means for calculating the consumption of anesthetic on the basis of the flow data obtained from the device (7) equipped with the flow measuring unit and the set value of the vaporization obtained from the reader device (9).

FIG. 3

## Description

The invention relates to a method in connection with anesthesia equipment, the anesteshesia equipment comprising a gas mixer, a connecting stud having a flow connection with the gas mixer, and a vaporizer, in which method the vaporizer is arranged in the connecting stud for vaporizing an anesthetic and the set value of the vaporizer is set by a mechanical regulator of the vaporizer. The invention further relates to an arrangement in connection with anesthesia equipment,

The invention relates to conventional anesthesia equipment utilising a rack of a particular kind called a Back Bar, onto which various units, such as vaporizers, are attached. Using the aforementioned Back Bar also enables a flow connection between the different units to be achieved.

An equipment solution of the Back Bar type has been known for decades in connection with anesthesia equipment. GB 1 385 670, for example, is a publication to disclose equipment of the Back Bar type.

The accuracy of mechanical regulators is directly proportional to a set value. The regulator is, for example, provided with a button equipped with an indicator point, which points to a scale. In principle, solutions of the Back Bar type operate in a completely satisfying manner, but the problem is, for example, that data on gas consumption and set values concerning such conventional vaporizer solutions cannot be advantageously supplied to the data system of for example a hospital, but difficult special procedures, for instance manual data feed or the like, must be employed if the data is to be used in the data system.

An object of the invention is to provide a method and an arrangement so as to enable disadvantages of the prior art to be eliminated. This is achieved by the invention. A method of the invention is characterized by measuring the flow of the vaporizer by a device equipped with a flow measuring unit, by reading the set value of the vaporizer on the basis of the position of a moving regulating member of the mechanical regulator, and by calculating the consumption of the anesthetic on the basis of measured flow data and the set value of vaporization obtained from the moving regulating member of the mechanical regulator. An arrangement of the invention, in turn, is characterized in that a device equipped with a flow measuring unit is arranged to measure the flow of the vaporizer, and that a reference scale corresponding to the regulation scale of the vaporizer is arranged in a moving regulating member of the mechanical regulator, and that the arrangement is equipped with a reader device arranged to read the reference scale, and that the arrangement comprises means for calculating the consumption of the anesthetic on the basis of flow data obtained from the device equipped with the flow measuring unit and the set value of the vaporization obtained from the reader device.

The main advantage of the invention is that the arrangement can be mounted afterwards in connection with substantially all conventional vaporizers in use, in which case data about gas consumption and settings can be incorporated in a hospital system advantageously. The arrangement of the invention also enables new mechanical vaporizers to be rendered electronic-like and thus the same advantages to be achieved in a simple way, as disclosed above in connection with the vaporizers in use at present. A further advantage of the arrangement of the invention is that it is simple, which means that the invention is inexpensive to introduce and use. The simplicity of the arrangement of the invention also results in reliable operation and little need for maintenance.

In the following, the invention will be explained in closer detail by means of the preferred embodiment described in the accompanying drawing, in which

Figure 1 is a schematic diagrammatic view of a flow arrangement in conventional anesthesia equipment,
Figure 2 is a schematic exploded view of essential elements of an arrangement of the invention, and
Figure 3 is a schematic view of the arrangement of the invention in use.

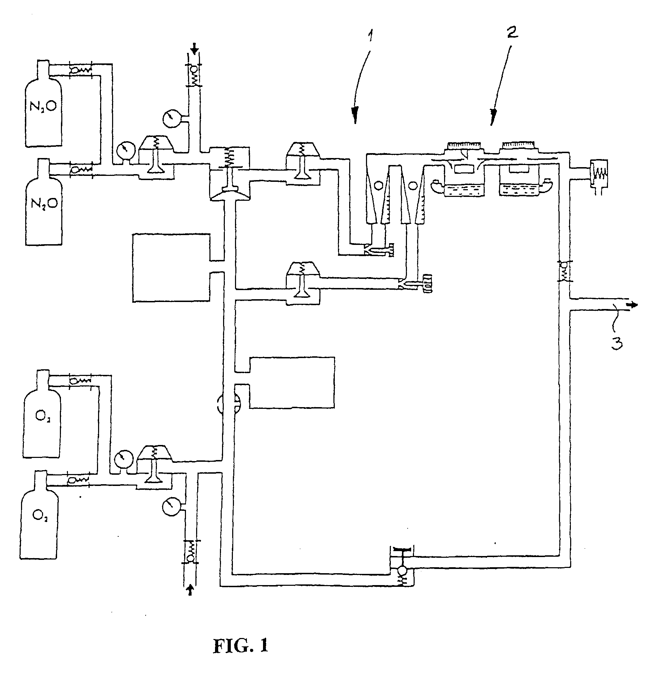

Figure 1 is a schematic diagrammatic view of a flow arrangement in a conventional anesthesia apparatus. In Figure 1, a gas mixer is denoted by reference number 1 and a vaporizer arrangement by reference number 2. A gas flow supplied to a patient is denoted by reference number 3.

The above-mentioned facts and the arrangement according to Figure 1 with its details as well as the operations of the arrangement and its details are obvious to one skilled in the art, so these facts will not be explained in closer detail in this connection.

The flow arrangement according to Figure 1 can be technically implemented in many ways. For instance, the aforementioned type solution called Back Bar and described in the aforementioned GB 1 385 670 can preferably be used in connection with the gas mixer 1 and a vaporizer.

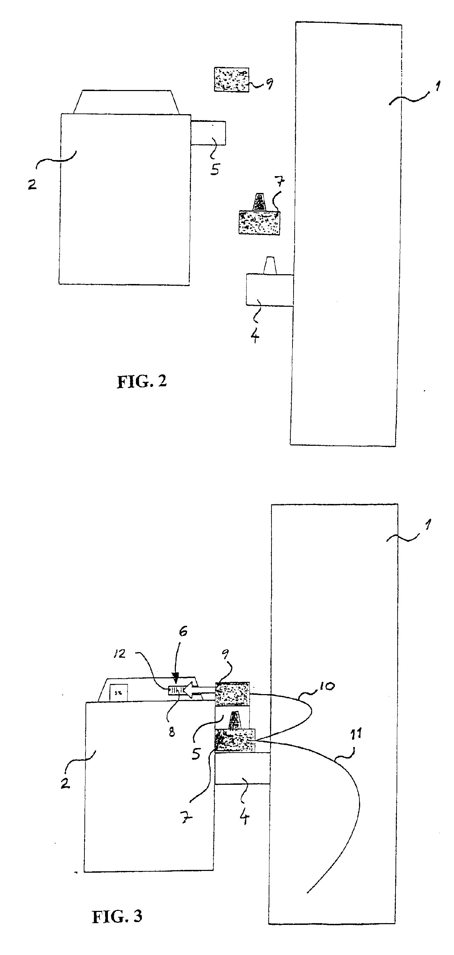

To be more precise, the invention thus relates to a vaporizer in an anesthesia apparatus, and to a vaporizer of the Back Bar type in particular. The invention will be described in closer detail in the accompanying Figures 2 and 3. For the sake of clarity, in Figures 2 and 3 the same reference numbers have the same significance as in Figure 1.

Figures 1 and 2 schematically show the gas mixer 1 and a vaporizer 2. A connecting stud for the vaporizer, i.e. a device called Back Bar enabling a flow connection to be established in a manner disclosed in GB 1 385 670, for example, is denoted by reference number 4. Naturally, the vaporizer 2 is equipped with a connecting member 5 compatible with the connecting stud 4. The vaporizer 2 is also equipped with means that enable the anesthetic to be fed into the vaporizer. These means are not shown in Figures 2 and 3. The above facts are obvious to one skilled in the art.

Also the operation of the vaporizer is obvious to one skilled in the art; consequently, this fact will not be described in closer detail in the connection. It is only noted in this connection that the vaporizer 2 comprises a mechanical regulator comprising a moving regulating member 6, for example a regulating wheel, and by changing the position of the regulating wheel the vaporizing percentage of the vaporizer can be regulated.

According to the underlying idea of the invention, a device 7 equipped with a flow measuring unit is arranged between the vaporizer 2 and the connecting stud 4, and in the example of the figures, the device 7 is arranged to measure the total flow travelling through the vaporizer 2. The device can be an appropriately shaped separate fitting piece, as shown in Figures 2 and 3. The flow measuring unit can preferably be comprised of means to measure a pressure difference over a laminar flow resistance, in other words a laminar element operating as a flow resistance is used and the pressure difference is measured over this element. Further possible ways include a hot-wire anenometer, flow propeller or measurement based on stagnation pressure. A reference scale 8 corresponding to the regulation scale of the vaporizer is arranged in the moving regulating member 6 of the mechanical regulator. The reference scale and the regulating scale of the vaporizer are thus similarly divided. The reference scale can be, for example, an optically readable scale, electrically readable scale or mechanically readable scale. An optically readdable scale can preferably be produced from, for example, a sticker equipped with a bar code and stuck onto the regulating wheel. An identifier 12 with e.g. a code 12 to identify the anesthetic used can also be arranged in the reference scale. Hence, the anesthetic being used can preferably be identified.

According to the underlying idea of the invention, the arrangement is further equipped with a reader device 9 arranged to read the reference scale in the moving regulating member 6, to read, for example, the position of the reference scale 8 and the position of the regulation member 6 simultaneously. The same reader device also identifies the identifier 12. The reader device 9 can be, for example, an optical device or an electrical device, etc. In Figure 3, the reading function of the reader device 9 is schematically shown by an arrow.

An arrangement of the invention further comprises means for calculating the consumption of anesthetic gas on the basis of the flow data obtained from the device 7 equipped with a flow measuring unit and the set value of the vaporization obtained from the reader device 9. The accuracy of the calculation naturally depends on the accuracy of the vaporizer itself, but the accuracy of the calculation is, however, accurate enough for monitoring purposes. The means for carrying out the aforementioned calculation can be any conventional means. The electronics necessary for data processing and calculating can be preferably arranged in the device 7. The necessary electric power is supplied by a cable or is obtained from a battery. The calculations can be carried out on the basis of the following equations, for example.

$$Vsc = Vmg*Cmg/100$$

$$Vane = Vsc/Xc,$$

wherein

Vsc = gas volume in millilitres of anesthetic consumed

Vane = volume in millilitres of liquid gas consumed

Xc = reduction factor characteristic of each anesthetic, the values for different anesthetics being approximately as follows: halothane 226, enflurane 96, isoflurane 195, sevoflurane 182, desflurane 207

Cmg = concentration of anesthetic in mixed gas (%)

Vmg = volume in millilitres of mixed gas consumed

As stated above, the anesthetic concentration Cmg of mixed gas is defined in the invention on the basis of the position of the regulating member 6 by the reader device 9, and the volume Vmg of mixed gas consumed is measured by the device 7. The anesthetic being used can be obtained from the code 12 by means of the reader device 9, and using this information, a correct coefficient Xc can be selected. Coefficient Xc can naturally be obtained otherwise, for example by manually feeding or producing a specific device 7 or calculation electronics only for each anesthetic. Gas consumption readings can be transferred, by cable or wirelessly, to a monitor or another device or directly to a data network. The cables are schematically shown by reference numbers 10 and 11 in Figure 3. As stated above, the cables can also be used to feed the necessary electric current.

Figures 2 and 3 show an arrangement of the invention wherein the device 7 equipped with a flow measuring device is a separate component. This, however, is not the only possible embodiment of the invention but the flow measuring unit can also be integrated into the connecting stud so as to be a part of the connecting stud 4. In such a case, only one flow measuring element is necessary although the connecting stud 4 might provide places for more, for example for three vaporizers. If the embodiment according to Figures 2 and 3 is used, each vaporizer needs a separate flow measuring unit of its own, in other words each vaporizer should have a device equipped with a flow measuring unit of its own.

The embodiments disclosed above are by no means intended to restrict the invention but the invention can be freely modified within the scope of the claims. It is thus obvious that an arrangement of the invention or the details thereof do not necessarily have to be identical with those disclosed in the figures, but also embodiments of other kind are possible.

**Claims**

1. A method in connection with anesthesia equipment comprising a gas mixer (1), a connecting stud (4) having a flow connection with the gas mixer, and a vaporizer (2), in which method the vaporizer (2) is arranged in the connecting stud (4) for vaporizing an anesthetic and the set value of the vaporizer is set by a mechanical regulator (6) of the vaporizer, **characterized** by measuring the flow of the vaporizer (2) by a device (7) equipped with a flow measuring unit, by reading the set value of the vaporizer on the basis of the position of a moving regulating member of the mechanical regulator (6), and by calculating the consumption of the anesthetic on the basis of measured flow data and the set value of vaporization obtained from the moving regulating member of the mechanical regulator (6).

2. A method as claimed in claim 1, **characterized** by measuring the flow from between the vaporizer (2) and the connecting stud (4).

3. A method as claimed in claim 1 or 2, **characterized** by measuring by the device (7) the total flow travelling through the vaporizer.

4. An arrangement in connection with anesthesia equipment comprising a gas mixer (1), a connecting stud (4) having a flow connection with the gas mixer, and a vaporizer (2) to be arranged in the connecting stud (4) for vaporizing an anesthetic, the vaporizer (2) being equipped with a mechanical regulator (6) for regulating the set value of the vaporizer, characterized in that a device (7) equipped with a flow measuring unit is arranged to measure the flow of the vaporizer (2), and that a reference scale (8) corresponding to the regulation scale of the vaporizer is arranged in a moving regulating member of the mechanical regulator (6), and that the arrangement is equipped with a reader device (9) arranged to read the reference scale (8), and that the arrangement comprises means for calculating the consumption of the anesthetic on the basis of the flow data obtained from the device (7) equipped with the flow measuring unit and the set value of the vaporization obtained from the reader device (9).

5. An arrangement as claimed in claim 4, **characterized** in that the device (7) equipped with the flow measuring unit is arranged between the vaporizer (2) and the connecting stud (4).

6. An arrangement as claimed in claim 4 or 5, **characterized** in that the device (7) equipped with the flow measuring unit is arranged to measure the total flow travelling through the vaporizer (2).

7. An arrangement as claimed in claim 5, **characterized** in that the device (7) equipped with the flow measuring unit is a separate component to be arranged in the connecting stud (4).

8. An arrangement as claimed in claim 5, **characterized** in that the device equipped with the flow measuring unit is integrated into the connecting stud (4).

9. An arrangement as claimed in claim 4, **characterized** in that the reference scale (8) is an optically readable scale.

10. An arrangement as claimed in claim 4, **characterized** in that the reference scale (8) is an electrically readable scale.

11. An arrangement as claimed in claim 4, **characterized** in that the reference scale (8) is a mechanically readable scale.

12. An arrangement as claimed in claim 9, 10 or 11, **characterized** in that the reference scale (8) is equipped with an identifier (12) to identify the anesthetic used.

13. An arrangement as claimed in claim 4, **characterized** in that the reader device is arranged to read the position of the reference scale.

**FIG. 1**

FIG. 2

FIG. 3